(19) Europäisches Patentamt
European Patent Office
Office européen des brevets

(11) Veröffentlichungsnummer: **0 328 766 A1**

(12) **EUROPÄISCHE PATENTANMELDUNG**

(21) Anmeldenummer: **88120962.1**

(22) Anmeldetag: **15.12.88**

(51) Int. Cl.4: **C07C 51/245 , C07C 63/333 , C07C 65/24 , C07C 149/40**

(30) Priorität: **22.12.87 DE 3743518**

(43) Veröffentlichungstag der Anmeldung: **23.08.89 Patentblatt 89/34**

(84) Benannte Vertragsstaaten: **CH DE FR GB IT LI**

(71) Anmelder: **BASF Aktiengesellschaft**
**Carl-Bosch-Strasse 38**
**D-6700 Ludwigshafen(DE)**

(72) Erfinder: **Neumann, Peter, Dr.**
**Poststrasse 28**
**D-6800 Mannheim 31(DE)**
Erfinder: **Eichenauer, Ulrich, Dr.**
**Paul-Ehrlich-Strasse 25 a**
**D-6000 Frankfurt 70(DE)**

(54) **Verfahren zur Herstellung von aromatischen Dicarbonsäuren.**

(57) Verfahren zur Herstellung von aromatischen Dicarbonsäuren der Formel

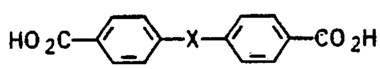

in der X für eine chemische Bindung, Sauerstoff oder Schwefel steht, durch Oxidation von Diacylverbindungen der Formel

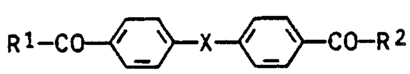

in der $R^1$ und $R^2$ unabhängig voneinander für gegebenenfalls substituiertes $C_1$-$C_4$-Alkyl stehen und X die obengenannte Bedeutung besitzt, mit Sauerstoff in Gegenwart eines Katalysators und eines Lösungsmittels bei einer Temperatur von 20 bis 250 °C.

EP 0 328 766 A1

## Verfahren zur Herstellung von aromatischen Dicarbonsäuren

Die vorliegende Erfindung betrifft ein neues Verfahren zur Herstellung von aromatischen Dicarbonsäuren durch Oxidation der entsprechenden Diacylverbindungen mittels Sauerstoff in Gegenwart eines Katalysators und eines Lösungsmittels.

Die Darstellung von Diphenyl-4,4′-dicarbonsäure aus 4,4′-Diacetyldiphenyl durch Haloform-Reaktion ist bekannt und beispielsweise in Ukr. Khim. Zh. Band 30, S. 938, 1964, beschrieben. Problematisch hierbei ist jedoch die Entstehung von Chloroform als Nebenprodukt sowie der Anfall größerer Mengen anorganischer Salze im Abwasser.

Es bestand daher die Aufgabe, ein Vefahren bereitzustellen, mittels dessen die durch Friedel-Crafts-Acylierung gut zugänglichen 4,4′-Diacylaryl-derivate unter Vermeidung der angesprochenen Probleme in die entsprechenden Dicarbonsäuren übergeführt werden können.

Es wurde nun gefunden, daß man aromatische Dicarbonsäuren der Formel I

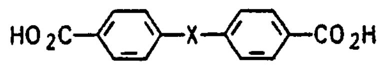 (I),

in der X für eine chemische Bindung, Sauerstoff oder Schwefel steht, vorteilhaft erhält, wenn man Diacylverbindungen der Formel II

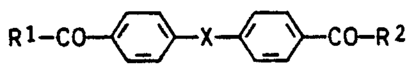 (II),

in der $R^1$ und $R^2$ gleich oder verschieden sind und unabhängig voneinander jeweils für gegebenenfalls substituiertes $C_1$-$C_4$-Alkyl stehen und X die obengenannte Bedeutung besitzt, mit Sauerstoff in Gegenwart eines Katalysators und eines Lösungsmittels bei einer Temperatur von 20 bis 250 °C oxidiert.

Aus der EP-A-216 279 ist bereits ein Verfahren bekannt, bei dem 5-Chlorthiophen-2-carbonsäure und 3-Chlorthiophen-2-carbonsäure durch Oxidation der entsprechenden 2-Acetylverbindungen mittels Sauerstoff in Gegenwart von Manganacetat oder Kobaltacetat als Katalysator in Eisessig hergestellt werden.

Im vorliegenden Fall war es nun überraschend, daß unter den Bedingungen des erfindungsgemäßen Verfahrens die zu erwartende Oxidation der aromatischen Kerne ausbleibt und somit keine dementsprechenden Nebenprodukte gebildet werden.

$R^1$ und $R^2$ stehen beispielsweise für Methyl, Ethyl, Propyl, Isopropyl, Butyl, oder Isobutyl.

Wenn die Reste $R^1$ und/oder $R^2$ substituiert sind, kommen als Substituenten z. B. Halogen, insbesondere Chlor, $C_1$-$C_4$-Alkoxy, wie Methoxy, Ethoxy, Propoxy, oder Butoxy, oder $C_1$-$C_4$-Alkanoyloxy, wie Formyloxy, Acetyloxy, Propionyloxy oder Butyryloxy in Betracht.

Solchermaßen substituierte Reste $R^1$ und $R^2$ sind insbesondere Chlormethyl, Methoxymethyl, Ethoxymethyl, Formyloxymethyl Acetyloxymethyl oder Propionylmethyl..

Bevorzugt werden solche acylierte aromatische Verbindungen der Formel II oxidiert in der $R^1$ und $R^2$ unabhängig voneinander Methyl oder Ethyl bedeutet.

Als Lösungsmittel kommen alle unter den Bedingungen des erfindungsgemäßen Verfahrens inerte organische Lösungsmittel in Betracht, wobei die Verwendung von $C_1$-$C_5$-Carbonsäuren bevorzugt ist. Beispielsweise seien Ameisensäure, Essigsäure, Propionsäure, Buttersäure, Valeriansäure oder Pivalinsäure genannt. Die Verwendung von Propionsäure als Lösungsmittel ist besonders bevorzugt.

Als Katalysatoren dienen beispielsweise Metallsalze, wobei insbesondere die Salze von Vanadium, Mangan, Eisen oder Kobalt zu nennen sind. Geeignete Anionen sind z. B. Phosphat, Sulfat, Halogenid, wie Fluorid, Chlorid oder Bromid, oder Carboxylate, wie Formiat, Acetat, Propionat oder Butyrat. Vorzugsweise verwendet man die jeweiligen Sulfate, Halogenide oder Acetate. Ganz besonders hervorzuheben sind diejenigen der genannten Metallacetate, in denen die Oxidationsstufe des jeweiligen Metalls + 2 beträgt.

In einer bevorzugten Ausführungsform verwendet man als Katalysatoren Mischungen der genannten Metallsalze, wobei insbesondere Kobalt- und Mangansalze als Mischungspartner zu nennen sind.

Die Menge der zu verwendenden Katalysatoren ist in einem weiten Bereich variierbar. Besonders zweckmäßig verwendet man die Katalysatoren in einer Menge von $5 \cdot 10^{-3}$ bis $5 \cdot 10^{-1}$ Mol Metallsalz, bezogen auf 1 Mol Verbindung II.

Die Reaktionstemperatur beträgt 50 bis 250 °C, wobei eine Temperatur von 90 °C bis zum Siedepunkt des Reaktionsgemischs bevorzugt ist. Man arbeitet dabei in der Regel bei einem Druck von 1 bis 25 bar,

wobei ein Reaktionsdruck von 1 bar bevorzugt ist.

Als Oxidationsmittel dient erfindungsgemäß Sauerstoff. Dabei kann der Sauerstoff in reiner Form oder in Mischung mit Inertgasen, beispielsweise Stickstoff, angewendet werden. Bevorzugt ist die Verwendung von Luft als Oxidationsmittel.

Das erfindungsgemäße Verfahren, das sowohl in kontinuierlicher als auch in diskontinuierlicher Arbeitsweise betrieben werden kann, wird zweckmäßig so durchgeführt, daß man in einem geeigneten Reaktor, z.B. in einem Flanschreaktor, die Diacylverbindung II, das Lösungsmittel und den Katalysator vorlegt und unter Rühren auf die erfindungsgemäße Temperatur erhitzt, wobei gleichzeitig Sauerstoff oder Luft durch das Reaktionsgemisch geleitet wird. Nach einer Reaktionszeit, die im allgemeinen 2 bis 20 Stunden beträgt, ist die Oxidation beendet und nach dem Abkühlen des Reaktionsgemischs wird dieses filtriert und der Filterrückstand mit Wasser gewaschen und getrocknet. Es ist auch möglich, das Reaktionsgemisch zunächst in Wasser zu geben und danach die Filtration vorzunehmen. Der behandelte Filterrückstand besteht im wesentlichen aus der aromatischen Dicarbonsäure I.

Die Diacylverbindungen der Formel II sind bekannt oder können nach an sich bekannten Methoden, beispielsweise durch Acylierung von Diphenyl oder Diphenylether und gegebenenfalls nachträglicher Modifizierung hergestellt werden (z. B. Chem. Ber. Band 120, S. 411, 1987).

Wie bereits ausgeführt ermöglicht das neue Verfahren überraschenderweise die Herstellung, der genannten Dicarbonsäuren in guter Ausbeute und ausgezeichneter Reinheit, wobei das Lösungsmittel mehrfach verwendet werden kann. Da keine unerwünschten Nebenprodukte anfallen und Sauerstoff oder Luft als Oxidationsmittel angewandt werden, ist die Synthese einfach durchführbar und umweltverträglich.

Die mittels des erfindungsgemäßen Verfahrens erhaltenen aromatischen Dicarbonsäuren der Formel I sind beispielsweise wertvolle Monomere zur Herstellung von thermotropen flüssigkristallinen Polymeren.

Die folgenden Beispiele sollen die Erfindung näher erläutern.

## Beispiel 1

In einem doppelwandigen 1 l-Flanschreaktor mit Intensivrührer und Stromstörer wurden 500 ml Eisessig, 47,6 g 4,4′-Diacetylbiphenyl, 0,8 g Kobalt(II)acetat und 12,0 g Mangan(II)acetat unter Rückfluß erhitzt und durch das Bodenventil mit Luft begast. Nach 18 Stunden wurde abgekühlt, abgesaugt, mit Wasser gewaschen und getrocknet. Man isolierte 43,1 g (89 %) Dicarbonsäure, Reinheit > 98 % (HPLC).

In analoger Weise werden die in der folgenden Tabelle aufgeführten Dicarbonsäuren erhalten. Die als Ausgangsprodukt dienenden Diacylverbindungen sind jeweils genannt.

Tabelle

| Bsp.-Nr. | Diacylverbindung | Dicarbonsäure |
|---|---|---|
| 2 | 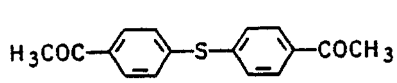 | 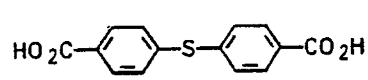 |
| 3 | 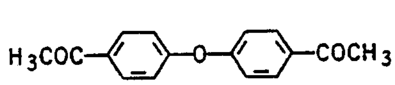 | 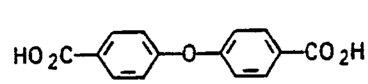 |
| 4 | 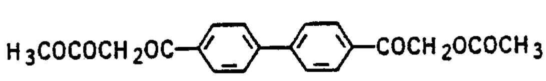 | 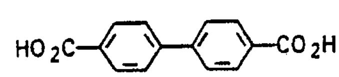 |

**Ansprüche**

1.Verfahren zur Herstellung von aromatischen Dicarbonsäuren der Formel I

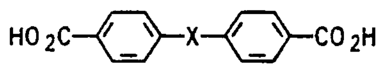 (I),

in der X für eine chemische Bindung, Sauerstoff oder Schwefel steht, dadurch gekennzeichnet, daß man Diacylverbindungen der Formel II

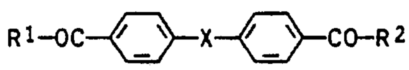 (II),

in der $R^1$ und $R^2$ gleich oder verschieden sind und unabhängig voneinander jeweils für gegebenenfalls substituiertes $C_1$-$C_4$-Alkyl stehen und X die obengenannte Bedeutung besitzt, mit Sauerstoff in Gegenwart eines Katalysators und eines Lösungsmittels bei einer Temperatur von 20 bis 250 °C oxidiert.

2.Verfahren gemäß Anspruch 1, dadurch gekennzeichnet, daß man die Oxidation in Gegenwart eines Salzes von Vanadium, Mangan, Eisen oder Kobalt als Katalysator durchführt.

3.Verfahren gemäß Anspruch 1, dadurch gekennzeichnet, daß man die Oxidation in Gegenwart einer $C_1$-$C_5$-Carbonsäure als Lösungsmittel durchführt.

Europäisches Patentamt

# EUROPÄISCHER RECHERCHENBERICHT

Nummer der Anmeldung

EP 88120962.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| D,A | EP - A1 - 0 216 279 (HOECHST)<br>* Ansprüche *<br>-- | 1-3 | C 07 C 51/245<br>C 07 C 63/333<br>C 07 C 65/24 |
| A | DE - C - 597 973 (I.G.FARBENINDUSTRIE)<br>* Gesamt *<br>---- | 1-3 | C 07 C 149/40 |
| | | | RECHERCHIERTE SACHGEBIETE (Int. Cl.4) |
| | | | C 07 C 51/00<br>C 07 C 63/00<br>C 07 C 65/00 |

Der vorliegende Recherchenbericht wurde für alle Patentansprüche erstellt.

| Recherchenort | Abschlußdatum der Recherche | Prüfer |
|---|---|---|
| WIEN | 22-03-1989 | HOFBAUER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82